# EUROPEAN PATENT SPECIFICATION

(11) **EP 4 011 425 B1**
(45) Date of publication and mention of the grant of the patent: **04.12.2024**
(21) Application number: 21213596.6
(22) Date of filing: 10.12.2021
(51) Int. Cl.: A61M 16/08, A61M 16/06, A61M 16/12, A61M 15/00

(54) **MULTIFUNCTION CONNECTOR FOR VENTILATION FACE MASKS**
MULTIFUNKTIONSVERBINDER FÜR BEATMUNGSMASKEN
CONNECTEUR MULTIFONCTION POUR MASQUES FACIAUX DE VENTILATION

(30) Priority: 11.12.2020 IT 202000030605
(43) Date of publication of application: 15.06.2022
(73) Proprietor: OLI - SISTEMAS SANITARIOS, S.A., 3800-314 Aveiro (PT)
(72) Inventor: SANTOS PEREIRA, Bruno, 3800-314 AVEIRO (PT); DE ALMEIDA MARTINS ANTUNES, Rogerio, 3800-314 AVEIRO (PT); DE AZEVEDO PINTO, Carlos Manuel, 3800-314 AVEIRO (PT); FONSECA MARTINS, Tiago Fernando, 3800-314 AVEIRO (PT); SOUSA GONCALVES LEONOR, José Tiago, 3800-314 AVEIRO (PT)
(74) Representative: Studio Torta S.p.A.

(56) References cited:
- EP-A1- 2 108 927
- WO-A1-2014/109749
- WO-A1-2017/014647
- US-A1- 2008 264 412

## Description

### CROSS-REFERENCE TO RELATED APPLICATIONS

This Patent Application claims priority from Italian Patent Application No. 102020000030605 filed on December 11, 2020.

### TECHNICAL FIELD

The present invention relates to the field of medical devices and in particular concerns a multifunction connector for connecting a non-invasive ventilation face mask to a ventilation system.

### BACKGROUND ART

Non-invasive ventilation (NIV) is a type of (mechanical) artificial ventilation used for patients with acute respiratory failure without the use of invasive techniques, such as orotracheal intubation or tracheostomy. The patient is fitted with a face mask connected to a ventilation system which supplies a gas flow to the mask and, therefore, to the patient.

The mask is connected to the ventilation system via a connector having, in general, an inlet connectable to a ventilation apparatus, an oxygen supply nozzle connectable to an oxygen supply pipe, and an outlet connectable to the mask.

In general, different connectors are required according to specific needs, or the use of different accessories in order to have at one's disposal the interfaces required by different patients. This requires the manufacture and management of various parts and components, with consequent complications and costs.

Furthermore, the need to select a specific connector and/or fit specific accessories may cause a loss of time while treating patients in an emergency.

Furthermore, known connectors that require the use of accessories and/or adapters are relatively bulky in size and weight and have protruding and/or irregular parts; when these connectors are placed on the patient's chest, as is common, they can therefore cause discomfort.

Furthermore, in most known connectors, the inlet and outlet ports consist of fixed nozzles, so that the flexible tubes connected to these nozzles remain perpendicular to the connector; however, this configuration may cause the tubes to bend, reducing the flowrate of fluid within them and potentially causing serious problems for the patient.

The document WO 2017/014647 A1 discloses a connector for connecting a non-invasive ventilation face mask to a ventilation system. The connector comprises an inlet connectable to the ventilation system and an outlet connectable to the ventilation mask. A connection portion of the outlet is shaped so as to be joined to a joint of the ventilation mask by means of a conical coupling. The connector further comprises an expiratory outlet and a pressure line port configured to be coupled to a pressure sampling line. T

The document WO 2014/109749 A1 discloses a multifunction connector for connecting an oxygen mask to a nebulizer. The multifunction connector includes a mask outlet port, a nebulizer inlet port, a metered-dose inhaler (MDI) port and an oxygen delivery port. T

The document US 2008/264412 A1 discloses a MDI ventilator assembly for use in a ventilator circuit for administering medication to a patient. The MDI ventilator assembly comprises a housing defining an interior space to allow an aerosolized drug to expand within the interior space before being inhaled by a patient. The housing defines an inhalation port for connection to a ventilator system, an exhalation port, a patient port for connection to an endotracheal or tracheostomy tube, a MDI receptacle for receiving a MDI container, a temperature probe port and a pressure probe port. T

### DISCLOSURE OF INVENTION

The present invention aims to solve the above-mentioned problems, in particular by providing a connector that simplifies and speeds up treatments requiring non-invasive ventilation of a patient in an emergency.

The present invention therefore relates to a multifunction connector for a ventilation face mask as defined in essential terms in the appended claim 1 and, in its additional features, in the dependent claims.

The connector of the invention consists of a multifunctional interface between a face mask and the non-invasive ventilation units, resulting in the following advantages with respect to the known technique.

The connector of the invention brings together several functionalities in one single device, allowing for a more rapid intervention when connecting patients to a ventilation system. The connector of the invention also allows for more stable treatment: by combining several functions, in particular allowing drugs to be administered via the MDI inlet, the connector of the invention avoids having to interrupt the ventilation circuit when drugs must be administered to the patient.

The connector of the invention also allows to reduce the risks of infection during treatment of respiratory diseases as it is not necessary to open the ventilation circuit to add any function.

The connector of the invention also allows to reduce the possibility of connection errors, given that the connector is provided with all the necessary accessories for the various functions.

The connector of the invention is advantageously provided with joint elements having different diameters chosen from various standards, so as to be easily and effectively connected to the various devices used in the healthcare sector.

In preferred embodiments, the oxygen inlet and the flow reading outlet are provided with rotating joints, so as to avoid excessive bending of the flexible tubes and to ensure the best air support for the patient.

Furthermore, the oxygen inlet and the flow reading outlet are provided with different removable joints that can only be fitted in the correct position, thus reducing the risk of incorrect connections.

Furthermore, the possibility of detaching the joints allows for an effective cleaning and sterilisation of all the parts of the connector.

In a version intended in particular for non-ventilated masks, the connector is additionally provided with an expiratory outlet for the emission of carbon dioxide. Advantageously, the expiratory outlet is positioned laterally on the body of the connector to prevent the contaminated air emitted by the patient from being directed towards the healthcare professionals and to reduce airborne contamination by optionally also allowing direct coupling of a HEPA filter.

The various functional elements are arranged so as to leave one side of the connector free of protrusions, offering greater comfort to the patient.

The connector of the invention can be manufactured in a simple and economical way and is compact and lightweight. In terms of logistics, it significantly reduces the number of parts in storage and in healthcare institutions will simplify ordering procedures.

### BRIEF DESCRIPTION OF THE DRAWINGS

Further characteristics and advantages of the present invention will become clear from the following description of a nonlimiting embodiment thereof, with reference to the figures of the accompanying drawings, in which:
- Figure 1 is a perspective view of a first embodiment of a multifunction connector for non-invasive ventilation face masks in accordance with the invention;
- Figure 2 is an exploded perspective view of the connector of figure 1;
- Figure 3 is a perspective view of the connector of figure 1, shown in use;
- Figure 4 is a perspective view of a component of the connector of figure 1;
- Figure 5 is a longitudinal sectional view of the connector of figure 1;
- Figure 6 is a perspective view, on an enlarged scale, of a further component of the connector of figure 1;
- Figure 7 is a cross-sectional view, with parts removed for clarity, of the connector of figure 1;
- Figure 8 is a perspective view of a second embodiment of the connector of the invention.

### BEST MODE FOR CARRYING OUT THE INVENTION

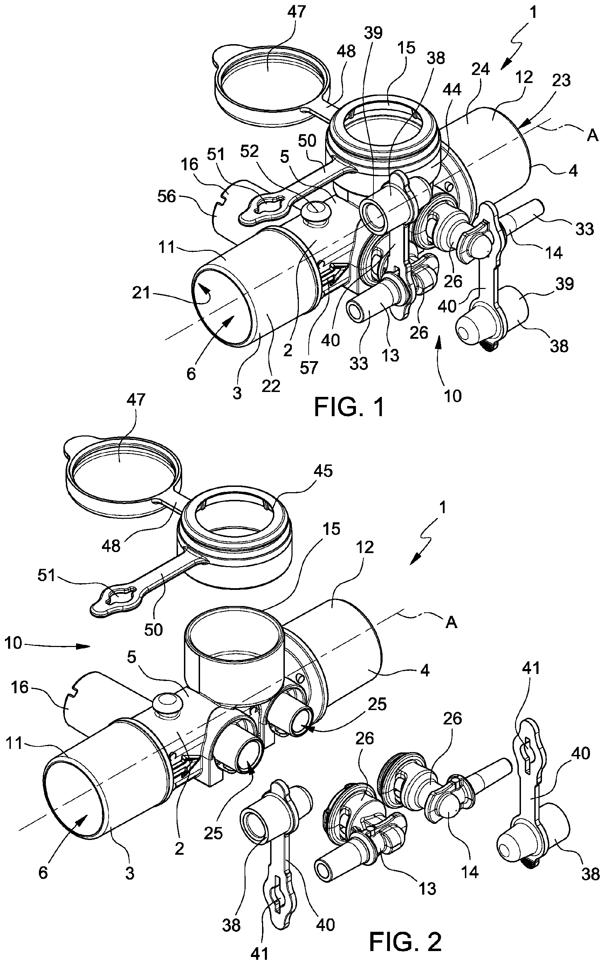

Figures 1, 2 and 3 designate, as a whole, with 1 a multifunction connector for a non-invasive ventilation face mask, usable to connect the mask to a ventilation system.

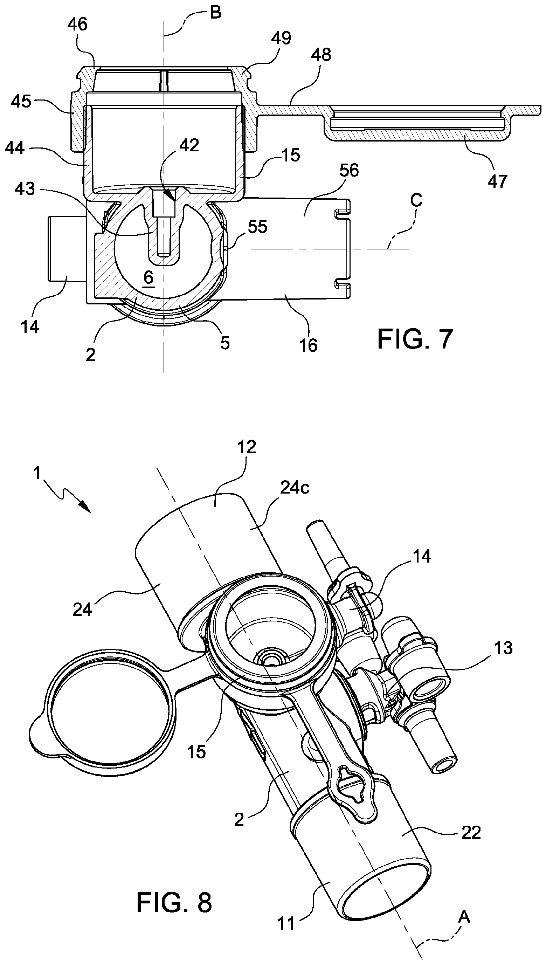

In particular, the connector 1 shown in figures 1 to 3 is intended for non-ventilated masks; further on, and with reference to figure 8, a variation specifically intended for ventilated masks is instead described.

The connector 1 comprises a base body 2, preferably consisting of a monolithic piece in polymer material, for example polycarbonate. Preferably, in accordance with common practice in the healthcare sector, the body 2 is made of a transparent or semi-transparent blue colored polymer material, to visually identify the type of connector intended for non-ventilated masks (whereas for ventilated masks a transparent or semi-transparent white or otherwise non-coloured material is used).

The body 2 is substantially a tubular body extending along a longitudinal axis A between two open axial ends 3, 4 and having a lateral wall 5 closed around the axis A and delimiting an internal duct 6.

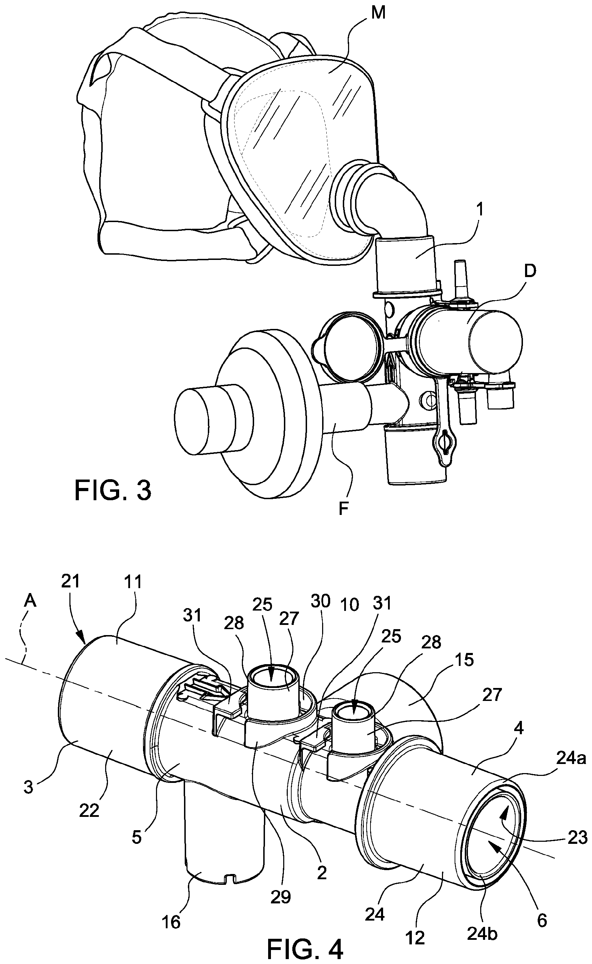

The connector 1 is provided with a plurality of functional connection elements 10 projecting from the body 2 and comprising in particular:
- an inlet 11 connectable to a ventilation system, in particular to a ventilator of a ventilation system;
- an outlet 12 connectable to a ventilation mask M (illustrated schematically in figure 3);
- an oxygen inlet 13;
- a flow reading outlet 14;
- an MDI inlet 15;
- an expiratory outlet 16.

With reference also to Figure 4, the inlet 11 is positioned at the end 3 of the body 2 and comprises an axial opening 21, positioned at the end 3 along the axis A and communicating with the duct 6; and a tubular connection portion 22 of the body 2 positioned around the opening 21 and shaped so as to be joined to a tube connected to the ventilation system, for example by means of a conical coupling.

The outlet 12 is positioned at the end 4 of the body 2 and comprises an axial opening 23, positioned at the end 4 and communicating with the duct 6 and substantially aligned along the axis A at the opening 21 of the inlet 11; and a tubular connection portion 24 of the body 2 positioned around the opening 23 and shaped so as to be joined to a joint of the mask M by means of a conical coupling. In the illustrated example, the connection portion 24 comprises a radially outer sleeve 24a and a radially inner sleeve 24b, coaxial around the axis A and radially spaced from one another.

For example, the connection portions 22, 24 define respective joint elements for conical couplings, for example, according to ISO 5356-1 standard.

For example (but not necessarily), the connection portion 22 of the inlet 11 defines a standard M22 male joint element according to ISO 5356-1 standard (male element 22 mm in diameter). In the connection portion 24 of the outlet 12, the radially outer sleeve 24a defines a standard M22 male joint element according to ISO 5356-1 standard (male element 22 mm in diameter), and the radially inner sleeve 24b defines a standard F15 female joint element according to ISO 5356-1 standard (female element 15 mm in diameter).

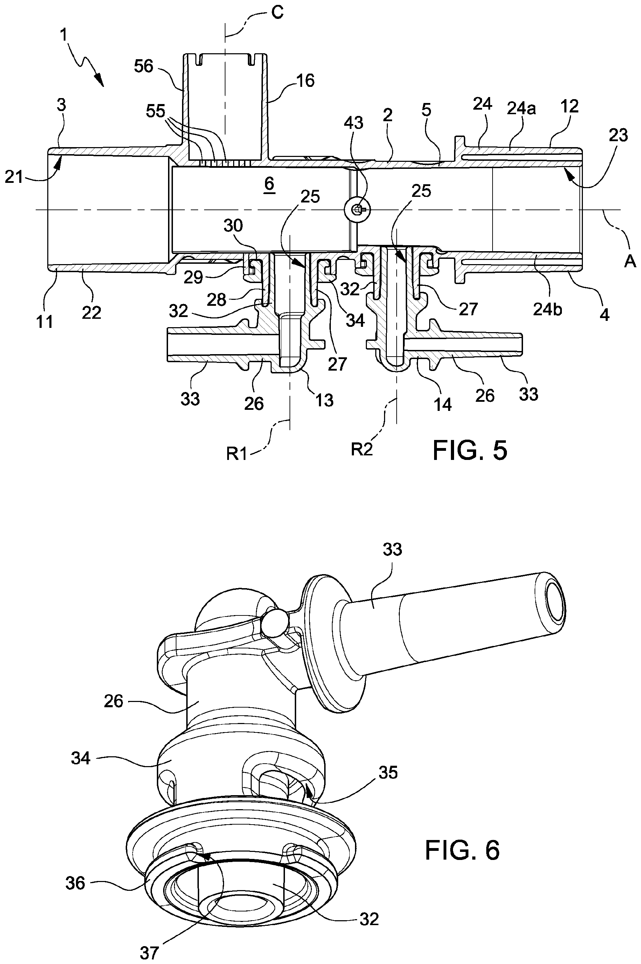

With reference also to Figures 5 and 6, the oxygen inlet 13 and the flow reading outlet 14 comprise respective radial openings 25 formed through the lateral wall 5 of the body 2 and communicating with the duct 6; and respective rotating joints 26 projecting from the lateral wall 5.

The joints 26 are connected in a rotatable manner to respective guide portions 27 which protrude integrally from the body 2 and are made integrally with the body 2; in particular, the joints 26 are rotatable with respect to the respective guide portions 27 around respective rotation axes R1, R2 perpendicular to the axis A.

In particular, each of the joints 26 is fastened to the respective guide portion 27 by means of a bayonet coupling so as to be axially fastened to the guide portion 27 and rotatable with respect thereto around the respective rotation axis R1, R2 and to be detachable from the guide portion 27 to allow removal of the joint 26 from the body 2.

Each guide portion 27 comprises an inner collar 28 protruding from the lateral wall 5 around the opening 25; and an outer collar 29 radially surrounding the collar 28 on the outside. The collars 28, 29 are concentric around the axis R1 or the axis R2 and delimit a groove 30 between them. The collar 29 has a portion of peripheral rim 31 bent radially inwards (i.e., towards the respective axis R1, R2).

Each of the joints 26 is substantially L-shaped and comprises a coupling portion 32, partially housed inside the collar 28 of the respective guide portion 27; a nozzle 33 connected to the coupling portion 32 and folded by 90° with respect thereto; and a radially outer annular portion 34, externally surrounding the coupling portion 32 and engaging the annular groove 30 of the respective guide portion 27.

The annular portion 34 has radial through slots 35 which communicate with an annular space defined between the coupling portion 32 and the annular portion 34 and which have the aim of facilitating cleaning and sterilisation.

The annular portion 34 is fastened to the collar 29 by means of an annular end edge 36 which engages the groove 30; the edge 36 axially fastens to the edge portion 31 of the collar 29 and has a circumferential break 37 to allow insertion into the groove 30.

In this manner, the joint 26 is detachable from the body 2 for operations of cleaning, sterilisation, etc.

The guide portions 27 are different from one another and the respective joints 26 are consequently different from one another and shaped to engage only the respective guide portion 27, thus avoiding assembly errors.

The oxygen inlet 13 and the flow reading outlet 14 are provided with respective plugs 38, preferably consisting of respective monolithic pieces in elastomeric material, for example TPE (thermoplastic elastomer).

Each of the plugs 38 comprises a cap 39, shaped so as to be fitted over an end portion of a nozzle 33 of the respective joint 26 to close the nozzle 33 in an airtight manner; and a joining band 40 projecting from a side of the cap 39 and having an end slit 41 fastened to the respective joint 26 so as to hold the plug 38 on the joint 26.

If the oxygen inlet 13 and/or the flow reading outlet 14 are not used, the respective nozzles 33 can be closed with the plugs 38, preventing infectious agents, dust and other external particles from entering the ventilation circuit and preventing air leaks which may reduce the flowrate required by the patient.

In the illustrated embodiment, the openings 25 are substantially side-by-side on the lateral wall 5 and therefore the oxygen inlet 13 and the flow reading outlet 14, as well as the respective joints 26, are side-by-side on the body 2; the joints 26 are rotatable around respective rotation axes R1, R2 parallel to each other.

With reference also to figure 7, the MDI inlet 15 comprises a radial opening 42 formed through the lateral wall 5 of the body 2 and communicating with the duct 6 through a nozzle 43 protruding into the duct 6; and a connection sleeve 44 formed integrally with the body 2 (therefore being part of the monolithic body 2) and extending from the lateral wall 5 around the opening 42 along an axis B.

In particular, the MDI inlet 15 is positioned on the lateral wall 5 in a position angularly staggered by 90° with respect to the oxygen inlet 13 and the flow reading outlet 14; the MDI inlet 15 extends along the B axis which is substantially perpendicular to the A axis and the axes R1, R2.

The MDI inlet 15 is configured to receive and engage a metered-dose inhaler (MDI) device D, for example in the form of a canister (shown schematically in figure 3).

Preferably, the MDI inlet 15 is provided with a flexible fastening ring 45 in elastomeric material, fitted on an end edge of the sleeve 44 and having a flexible radially inner annular lip 46, which makes the MDI inlet 15 adaptable to different diameters of the metered-dose inhaler devices (typically in canister form) and prevents the entry of external agents by adhering to the lateral wall of the canister. The lip 46 cooperates, in use, with an outer lateral surface of the metered-dose inhaler device D housed in the sleeve 44.

The MDI inlet 15 is also provided with a plug 47 to close the MDI inlet 15.

The plug 47 is preferably made integrally with the fastening ring 45 to form a monolithic piece in elastomeric material, for example TPE (thermoplastic elastomer).

The plug 47 is connected to the fastening ring 45 by a flexible tongue 48 and is shaped so as to engage with a peripheral rim 49 of the fastening ring 45 to close the fastening ring 45 and therefore the sleeve 44.

Advantageously, the plug 47 also comprises a flexible strap 50 extending laterally from the fastening ring 45 and is provided with an end slit 51 to be engaged with a pin 52 projecting from the lateral wall 5 of the body 2, so as to retain the assembly formed by the fastening ring 45 and the plug 47 on the body 2.

The expiratory outlet 16 is used to expel from the body 2 a flow of air exhaled by the patient (flow of carbon dioxide) and is connectable to a filter F (schematically illustrated in figure 3), for example a HEPA filter.

In particular, the expiratory outlet 16 comprises a set of radial holes 55 formed through the lateral wall 5 of the body 2 and communicating with the duct 6; and a connection sleeve 56, formed integrally with the body 2 (therefore being part of the monolithic body 2) and extending from the lateral wall 5 along an axis C so as to surround the set of holes 55.

The sleeve 56 is shaped so as to define a joint element, for example, a joint element according to ISO 5356-1 standard (in particular, an M15 male element 15 mm in diameter), connectable to an external element, for example a HEPA filter.

In the preferred embodiment illustrated, the expiratory outlet 16 is positioned on the lateral wall 5 in a position angularly staggered by 90° with respect to the MDI inlet 15 and opposite to the oxygen inlet 13 and the flow reading outlet 14; the expiratory outlet 16 extends along an axis C which is substantially perpendicular to the axis A and to the axis B and parallel to the axes R1, R2.

The oxygen inlet 13, the flow reading outlet 14, the MDI inlet 15 and the expiratory outlet 16 are arranged on three sides of the body 2, leaving a fourth side devoid of protrusions, i.e. where no functional elements 10 are positioned which protrude from that side of the body 2 (meaning as sides of the body 2 respective lateral portions of the body 2 generically facing respective planes parallel to the axis A and orthogonal to each other).

The body 2 therefore has a side devoid of protrusions (i.e. protruding functional elements 10), which can be placed on or beside the patient's body without causing discomfort.

Advantageously, one or more arrows 57 (figure 1) are provided on the body 2 indicating the direction of the ventilation flow towards the patient, to facilitate the use of the connector 1 by providing the healthcare professionals with an immediate indication of the correct mounting direction of the connector 1 with respect to the patient's face mask.

For example, the body 2 has a pair of arrows 57 arranged on opposite sides of the body 2, preferably raised or engraved on an outer lateral surface of the body 2.

A version of the connector of the invention specifically intended for use with ventilated masks is illustrated in figure 8, in which details similar or the same as those already described are indicated by the same numbers.

Also in this embodiment, the connector 1 comprises a base body 2, preferably consisting of a monolithic piece of polymeric material, for example polycarbonate. Preferably, in accordance with common practice in the healthcare sector, the body 2 is made of transparent or semi-transparent non-coloured (substantially white) polymer material, to visually identify the type of connector intended for ventilated masks.

With respect to what has been described above, the connector 1 differs in that: it is devoid of the expiratory outlet 16 (the body 2 therefore has no further lateral holes for expelling air/CO2); and the outlet 12 for connection to the mask has a connection portion 24 formed by a single sleeve 24c instead of a pair of coaxial sleeves.

For example, but not necessarily, the sleeve 24c defines a standard F22 female connecting element according to ISO 5356-1 standard (whereas in the connector for non-ventilated masks, the connection portion 24 comprises a radially outer sleeve 24a defining a standard M22 male connecting element, and a radially inner sleeve 24b defining a standard F15 female connecting element).

As already described above, therefore, also in this embodiment, the connector 1 is provided with:
- an inlet 11 connectable to a ventilation system;
- an outlet 12 connectable to a ventilation mask;
- an oxygen inlet 13
- a flow reading outlet 14;
- an MDI inlet 15.

It is also clear that further modifications and variations can be made to the connector described and illustrated herein, which do not depart from the scope of the claims attached herein.

## Claims

1. A multifunction connector (1) for non-invasive ventilation face masks, configured for connecting a non-invasive ventilation face mask to a ventilation system; comprising a substantially tubular base body (2) extending along a longitudinal axis (A) between two opposite open axial ends (3, 4) and having a lateral wall (5) closed around the axis (A) and delimiting an internal duct (6); the connector (1) being provided with a plurality of functional connection elements (10) projecting from the body (2) and comprising:
- an inlet (11) connectable to a ventilation system and an outlet (12) connectable to a ventilation mask, positioned at respective opposite axial ends (3, 4) of the body (2) and comprising respective tubular connection portions (22, 24) of the body (2); the connection portion (24) of the outlet (12) being shaped so as to be joined to a joint of the ventilation mask by means of a conical coupling;
- an oxygen inlet (13) connectable to an oxygen supply pipe;
- a flow reading outlet (14) connectable to a flow measuring device;
- an MDI inlet (15) configured to receive and engage a metered-dose inhaler device.

2. A connector according to claim 1, wherein the oxygen inlet (13) and the flow reading outlet (14) comprise respective radial openings (25) formed through the lateral wall (5) of the body (2) and communicating with the duct (6); and respective rotating joints (26) connected in a rotatable manner to respective guide portions (27) which protrude integrally from the body (2), so as to be rotatable with respect to said respective guide portions (27) around respective rotation axes (R1, R2) perpendicular to the axis (A).

3. A connector according to claim 2, wherein each rotating joint (26) is fastened to the respective guide portion (27) by means of a bayonet coupling so as to be axially fastened to the guide portion (27) and rotatable with respect thereto around the respective rotation axis (R1, R2), and to be detachable from the guide portion (27) to allow removal of the joint (26) from the body (2).

4. A connector according to claim 3, wherein the guide portions (27) are different from each other and the respective joints (26) are shaped so as to engage only the respective guide portion (27), thus avoiding assembly errors.

5. A connector according to one of claims 2 to 4, wherein each joint (26) is substantially L-shaped and comprises a coupling portion (32), partially housed inside a collar (28) of the respective guide portion (27); a nozzle (33) connected to the coupling portion (32) and folded by 90° with respect thereto; and a radially outer annular portion (34), externally surrounding the coupling portion (32) and engaging an annular groove (30) formed on the body (2); and wherein the annular portion (34) has radial through slots (35) communicating with an annular space defined between the coupling portion (32) and the annular portion (34).

6. A connector according to one of the preceding claims, wherein the oxygen inlet (13) and the flow reading outlet (14) are provided with respective plugs (38), consisting of respective monolithic pieces in elastomeric material, for example TPE; each of the plugs (38) comprising a cap (39), shaped to fit over an end portion of the respective joint (26); and a joining band (40) projecting from a side of the cap (39) and having an end slit (41) fastened to the respective joint (26) so as to hold the plug (38) on the joint (26).

7. A connector according to one of the preceding claims, wherein the oxygen inlet (13) and the flow reading outlet (14) are arranged side-by-side on the body (2).

8. A connector according to one of the preceding claims, wherein the MDI inlet (15) comprises a radial opening (42) formed through the lateral wall (5) of the body (2) and communicating with the duct (6) via a nozzle (43) projecting in the duct (6); and a connection sleeve (44), formed integrally with the body (2) and extending from the lateral wall (5) around said opening (42) .

9. A connector according to claim 8, wherein the MDI inlet (15) is provided with a flexible fastening ring (45) in elastomeric material, fitted on an end edge of the connection sleeve (44) and having a flexible radially inner annular lip (46) cooperating, in use, with an outer lateral surface of a metered-dose inhaler device housed in the connection sleeve (44) .

10. A connector according to claim 9, wherein the MDI inlet (15) is provided with a plug (47) for closing the MDI inlet (15), integrally formed with the fastening ring (45) to form a monolithic piece in elastomeric material and connected to the fastening ring (45) by a flexible tongue (48) and shaped to engage a peripheral rim (49) of the fastening ring (45) to close the fastening ring (45) and thus the connection sleeve (44); the plug (47) further comprising a flexible strap (50) extending laterally from the fastening ring (45) and is provided with an end slit (51) engaging a pin (52) projecting from the lateral wall (5) of the body (2) .

11. A connector according to one of the preceding claims, wherein the MDI inlet (15) is positioned on the lateral wall (5) of the body (2) in a position angularly staggered by 90° with respect to the oxygen inlet (13) and the flow reading outlet (14) .

12. A connector according to one of the preceding claims, wherein the connection functional elements (10) comprise also an expiratory outlet (16), configured for expulsion of an air flow from the body (2) and for connection to a filter.

13. A connector according to claim 12, wherein the expiratory outlet (16) comprises a set of radial holes (55) formed through the lateral wall (5) of the body (2) and communicating with the duct (6); and a connection sleeve (56), integrally formed with the body (2) and extending from the lateral wall (5) to surround the set of holes (55) .

14. A connector according to claim 12 or 13, wherein the expiratory outlet (16) is positioned on the lateral wall (5) in a position angularly staggered by 90° with respect to the MDI inlet (15) and opposite to the oxygen inlet (13) and the flow reading outlet (14).

15. A connector according to one of claims 12 to 14, wherein the oxygen inlet (13), the flow reading outlet (14), the MDI inlet (15) and the expiratory outlet (16) are arranged on three sides of the body (2), leaving a fourth side substantially without projections defined by functional elements (10).

16. A connector according to one of the preceding claims, wherein the body (2) is provided with one or more arrows (57) indicating the direction of the ventilation flow towards the patient, so as to provide a visual indication of the correct mounting direction of the connector (1) with respect to the mask.

17. A connector according to one of the preceding claims, wherein the body (2) consists of a monolithic piece in polymeric material, for example polycarbonate, and is made of a blue colored polymer material at least partially transparent, if the connector is designed for non-ventilated masks; or of a white or non-colored polymer material at least partially transparent, if the connector is designed for ventilated masks.

## Patentansprüche

1. Multifunktionsverbinder (1) für nichtinvasive Beatmungsgesichtsmasken, der zum Verbinden einer nicht-invasiven Beatmungsgesichtsmaske mit einem Beatmungssystem konfiguriert ist; umfassend einen im Wesentlichen rohrförmigen Grundkörper (2), der sich entlang einer Längsachse (A) zwischen zwei gegenüberliegenden offenen axialen Enden (3, 4) erstreckt und eine Seitenwand (5) aufweist, die um die Achse (A) herum geschlossen ist und einen inneren Kanal (6) begrenzt; wobei der Verbinder (1) mit einer Vielzahl von Verbindungsfunktionselementen (10) versehen ist, die von dem Körper (2) vorstehen und umfassen:
- einen Einlass (11), der mit einem Beatmungssystem verbunden werden kann, und einen Auslass (12), der mit einer Beatmungsmaske verbunden werden kann, die an jeweiligen gegenüberliegenden axialen Enden (3, 4) des Körpers (2) angeordnet sind und jeweilige rohrförmige Verbindungsabschnitte (22, 24) des Körpers (2) umfassen; wobei der Verbindungsabschnitt (24) des Auslasses (12) so geformt ist, dass er mit einer Verbindung der Beatmungsmaske mittels einer konischen Kupplung verbunden werden kann;
- einen Sauerstoffeinlass (13), der mit einer Sauerstoffzufuhrleitung verbunden werden kann;
- einen Durchflussmessauslass (14), der mit einem Durchflussmessgerät verbunden werden kann;
- einen MDI-Einlass (15), der so konfiguriert ist, dass er ein Dosierinhalationsgerät aufnimmt und in Eingriff bringt.

2. Verbinder nach Anspruch 1, wobei der Sauerstoffeinlass (13) und der Durchflussmessauslass (14) jeweilige radiale Öffnungen (25), die durch die Seitenwand (5) des Körpers (2) hindurch ausgebildet sind und mit dem Kanal (6) in Verbindung stehen, und jeweilige Drehgelenke (26) umfassen, die drehbar mit jeweiligen Führungsabschnitten (27) verbunden sind, die einstückig aus dem Körper (2) vorstehen, so dass sie in Bezug auf die jeweiligen Führungsabschnitte (27) um jeweilige Drehachsen (R1, R2) senkrecht zur Achse (A) drehbar sind.

3. Verbinder nach Anspruch 2, wobei jedes Drehgelenk (26) an dem jeweiligen Führungsabschnitt (27) mittels einer Bajonettkupplung befestigt ist, um axial an dem Führungsabschnitt (27) befestigt und in Bezug auf diesen um die jeweilige Drehachse (R1, R2) drehbar zu sein, und um von dem Führungsabschnitt (27) lösbar zu sein, um das Entfernen des Gelenks (26) von dem Körper (2) zu ermöglichen.

4. Verbinder nach Anspruch 3, wobei die Führungsabschnitte (27) voneinander verschieden sind und die jeweiligen Gelenke (26) so geformt sind, dass sie nur in den jeweiligen Führungsabschnitt (27) eingreifen, wodurch Montagefehler vermieden werden.

5. Verbinder nach einem der Ansprüche 2 bis 4, wobei jedes Gelenk (26) im wesentlichen L-förmig ist und einen Kupplungsabschnitt (32), der teilweise innerhalb eines Kragens (28) des jeweiligen Führungsabschnitts (27) untergebracht ist, eine Düse (33), die mit dem Kupplungsabschnitt (32) verbunden und in Bezug auf diesen um 90° gefaltet ist, und einen radial äußeren ringförmigen Abschnitt (34) umfasst, der den Kupplungsabschnitt (32) außen umgibt und in eine auf dem Körper (2) ausgebildete ringförmige Nut (30) eingreift; und wobei der ringförmige Abschnitt (34) radiale Durchgangsschlitze (35) aufweist, die mit einem zwischen dem Kupplungsabschnitt (32) und dem ringförmigen Abschnitt (34) definierten ringförmigen Raum in Verbindung stehen.

6. Verbinder nach einem der vorstehenden Ansprüche, wobei der Sauerstoffeinlass (13) und der Durchflussmessauslass (14) mit jeweiligen Stopfen (38) versehen sind, die aus jeweiligen monolithischen Stücken aus elastomerem Material, beispielsweise TPE, bestehen; wobei jeder der Stopfen (38) eine Kappe (39) umfasst, die so geformt ist, dass sie über einen Endabschnitt des jeweiligen Gelenks (26) passt; und ein Verbindungsband (40), das von einer Seite der Kappe (39) vorsteht und einen Endschlitz (41) aufweist, der an dem jeweiligen Gelenk (26) befestigt ist, um den Stopfen (38) auf dem Gelenk (26) zu halten.

7. Verbinder nach einem der vorstehenden Ansprüche, wobei der Sauerstoffeinlass (13) und der Durchflussmessauslass (14) nebeneinander am Körper (2) angeordnet sind.

8. Verbinder nach einem der vorstehenden Ansprüche, wobei der MDI-Einlass (15) eine radiale Öffnung (42), die durch die Seitenwand (5) des Körpers (2) hindurch ausgebildet ist und mit dem Kanal (6) über eine in den Kanal (6) hineinragende Düse (43) in Verbindung steht, und eine Verbindungshülse (44) umfasst, die einstückig mit dem Körper (2) ausgebildet ist und sich von der Seitenwand (5) um die Öffnung (42) herum erstreckt.

9. Verbinder nach Anspruch 8, wobei der MDI-Einlass (15) mit einem flexiblen Befestigungsring (45) aus elastomerem Material versehen ist, der an einer Endkante der Verbindungshülse (44) angebracht ist und eine flexible, radial innere, ringförmige Lippe (46) aufweist, die im Gebrauch mit einer äußeren Seitenfläche einem in der Verbindungshülse (44) untergebrachten Dosierinhalationsgerät zusammenwirkt.

10. Verbinder nach Anspruch 9, wobei der MDI-Einlass (15) mit einem Stopfen (47) zum Verschließen des MDI-Einlasses (15) versehen ist, der einstückig mit dem Befestigungsring (45) ausgebildet ist, um ein monolithisches Teil aus elastomerem Material zu bilden, und der mit dem Befestigungsring (45) durch eine flexible Zunge (48) verbunden ist und so geformt ist, dass er in einen Umfangsrand (49) des Befestigungsrings (45) eingreift, um den Befestigungsring (45) und damit die Verbindungshülse (44) zu schließen; wobei der Stopfen (47) ferner ein flexibles Band (50) aufweist, das sich seitlich von dem Befestigungsring (45) erstreckt und mit einem Endschlitz (51) versehen ist, der in einen Stift (52) eingreift, der von der Seitenwand (5) des Körpers (2) vorsteht.

11. Verbinder nach einem der vorstehenden Ansprüche, wobei der MDI-Einlass (15) an der Seitenwand (5) des Körpers (2) in einer Position positioniert ist, die in Bezug auf den Sauerstoffeinlass (13) und den Durchflussmessauslass (14) um 90° winkelversetzt ist.

12. Verbinder nach einem der vorstehenden Ansprüche, wobei die Verbindungsfunktionselemente (10) auch einen Ausatmungsauslass (16) umfassen, der zum Ausstoßen eines Luftstroms aus dem Körper (2) und zum Anschluss an einen Filter konfiguriert ist.

13. Verbinder nach Anspruch 12, wobei der Ausatmungsauslass (16) einen Satz von radialen Löchern (55), die durch die Seitenwand (5) des Körpers (2) hindurch ausgebildet sind und mit dem Kanal (6) in Verbindung stehen, und eine Verbindungshülse (56) umfasst, die einstückig mit dem Körper (2) ausgebildet ist und sich von der Seitenwand (5) aus erstreckt, um den Satz von Löchern (55) zu umgeben.

14. Verbinder nach Anspruch 12 oder 13, wobei der Ausatmungsauslass (16) an der Seitenwand (5) in einer Position angeordnet ist, die in Bezug auf den MDI-Einlass (15) um 90° winkelversetzt ist und dem Sauerstoffeinlass (13) und dem Durchflussmessauslass (14) gegenüberliegt.

15. Verbinder nach einem der Ansprüche 12 bis 14, wobei der Sauerstoffeinlass (13), der Durchflussmessauslass (14), der MDI-Einlass (15) und der Ausatmungsauslass (16) auf drei Seiten des Körpers (2) angeordnet sind und eine vierte Seite im Wesentlichen ohne Vorsprünge verbleibt, die durch Funktionselemente (10) definiert sind.

16. Verbinder nach einem der vorstehenden Ansprüche, wobei der Körper (2) mit einem oder mehreren Pfeilen (57) versehen ist, die die Richtung des Beatmungsstroms zum Patienten hin anzeigen, um so eine visuelle Anzeige der korrekten Montagerichtung des Verbinders (1) in Bezug auf die Maske bereitzustellen.

17. Verbinder nach einem der vorstehenden Ansprüche, wobei der Körper (2) aus einem monolithischen Stück aus Polymermaterial, beispielsweise Polycarbonat, besteht und aus einem blau gefärbten, zumindest teilweise transparenten Polymermaterial hergestellt ist, wenn der Verbinder für nicht belüftete Masken ausgelegt ist, oder aus einem weißen oder nicht gefärbten, zumindest teilweise transparenten Polymermaterial, wenn der Verbinder für belüftete Masken ausgelegt ist.

## Revendications

1. Connecteur multifonction (1) pour masques faciaux de ventilation non invasifs, configuré pour relier un masque facial de ventilation non invasif à un système de ventilation ; comprenant un corps de base sensiblement tubulaire (2) s'étendant le long d'un axe longitudinal (A) entre deux extrémités axiales ouvertes opposées (3, 4) et ayant une paroi latérale (5) fermée autour de l'axe (A) et délimitant un conduit interne (6) ; le connecteur (1) étant pourvu d'une pluralité d'éléments de connexion fonctionnels (10) faisant saillie à partir du corps (2) et comprenant :
- une entrée (11) pouvant être reliée à un système de ventilation et une sortie (12) pouvant être reliée à un masque de ventilation, positionnées aux extrémités axiales opposées (3, 4) du corps (2) et comprenant des parties de connexion tubulaires (22, 24) du corps (2) ; la partie de connexion (24) de la sortie (12) étant formée de manière à être reliée à un joint du masque de ventilation au moyen d'un accouplement conique ;
- une entrée d'oxygène (13) pouvant être reliée à un tuyau d'alimentation en oxygène ;
- une sortie de lecture de débit (14) pouvant être reliée à un dispositif de mesure de débit;
- une entrée d'aérosol-doseur (MDI) (15) configurée pour recevoir et engager un aérosol-doseur.

2. Connecteur selon la revendication 1, dans lequel l'entrée d'oxygène (13) et la sortie de lecture de débit (14) comprennent des ouvertures radiales respectives (25) formées à travers la paroi latérale (5) du corps (2) et communiquant avec le conduit (6) ; et des joints rotatifs respectifs (26) reliés de manière rotative à des parties de guidage respectives (27) qui font saillie intégralement du corps (2), de manière à pouvoir tourner par rapport auxdites parties de guidage respectives (27) autour d'axes de rotation respectifs (R1, R2) perpendiculaires à l'axe (A).

3. Connecteur selon la revendication 2, dans lequel chaque joint rotatif (26) est fixé à la partie de guidage respective (27) au moyen d'un accouplement à baïonnette de manière à être fixé axialement à la partie de guidage (27) et à pouvoir tourner par rapport à celle-ci autour de l'axe de rotation respectif (R1, R2), et à pouvoir être détaché de la partie de guidage (27) pour permettre le retrait du joint (26) du corps (2) .

4. Connecteur selon la revendication 3, dans lequel les parties de guidage (27) sont différentes les unes des autres et les joints respectifs (26) sont façonnés de manière à n'engager que la partie de guidage respective (27), évitant ainsi les erreurs d'assemblage.

5. Connecteur selon l'une des revendications 2 à 4, dans lequel chaque joint (26) est sensiblement en forme de L et comprend une partie de couplage (32), partiellement logée à l'intérieur d'un collier (28) de la partie de guidage respective (27) ; une buse (33) reliée à la partie de couplage (32) et pliée à 90° par rapport à celle-ci ; et une partie annulaire radialement extérieure (34), entourant extérieurement la partie de couplage (32) et s'engageant dans une rainure annulaire (30) formée sur le corps (2) ; et dans laquelle la partie annulaire (34) a des fentes radiales traversantes (35) communiquant avec un espace annulaire défini entre la partie de couplage (32) et la partie annulaire (34).

6. Connecteur selon l'une des revendications précédentes, dans lequel l'entrée d'oxygène (13) et la sortie de lecture de débit (14) sont pourvues de bouchons respectifs (38), constitués de pièces monolithiques respectives en matériau élastomère, par exemple TPE ; chacun des bouchons (38) comprenant un capuchon (39), formé pour s'adapter à une partie d'extrémité du joint respectif (26) ; et une bande d'assemblage (40) faisant saillie d'un côté du capuchon (39) et ayant une fente d'extrémité (41) fixée au joint respectif (26) de manière à maintenir le bouchon (38) sur le joint (26).

7. Connecteur selon l'une des revendications précédentes, dans lequel l'entrée d'oxygène (13) et la sortie de lecture de débit (14) sont disposées côte à côte sur le corps (2).

8. Connecteur selon l'une des revendications précédentes, dans lequel l'entrée MDI (15) comprend une ouverture radiale (42) formée à travers la paroi latérale (5) du corps (2) et communiquant avec le conduit (6) par l'intermédiaire d'une buse (43) faisant saillie dans le conduit (6) ; et un manchon de connexion (44), formé intégralement avec le corps (2) et s'étendant à partir de la paroi latérale (5) autour de ladite ouverture (42).

9. Connecteur selon la revendication 8, dans lequel l'entrée MDI (15) est pourvue d'un anneau de fixation flexible (45) en matériau élastomère, monté sur un bord d'extrémité du manchon de connexion (44) et ayant une lèvre annulaire flexible radialement intérieure (46) coopérant, lors de l'utilisation, avec une surface latérale extérieure d'un aérosol-doseur logé dans le manchon de connexion (44).

10. Connecteur selon la revendication 9, dans lequel l'entrée MDI (15) est pourvue d'un bouchon (47) pour fermer l'entrée MDI (15), formé intégralement avec l'anneau de fixation (45) pour former une pièce monolithique en matériau élastomère et relié à l'anneau de fixation (45) par une languette flexible (48) et façonnée pour s'engager dans un bord périphérique (49) de l'anneau de fixation (45) pour fermer l'anneau de fixation (45) et donc le manchon de connexion (44) ; le bouchon (47) comprenant en outre une bande flexible (50) s'étendant latéralement à partir de l'anneau de fixation (45) et pourvue d'une fente d'extrémité (51) s'engageant dans une broche (52) faisant saillie de la paroi latérale (5) du corps (2) .

11. Connecteur selon l'une des revendications précédentes, dans lequel l'entrée MDI (15) est positionnée sur la paroi latérale (5) du corps (2) dans une position décalée angulairement de 90° par rapport à l'entrée d'oxygène (13) et à la sortie de lecture de débit (14).

12. Connecteur selon l'une des revendications précédentes, dans lequel les éléments fonctionnels de connexion (10) comprennent également une sortie d'expiration (16), configurée pour l'expulsion d'un flux d'air du corps (2) et pour la connexion à un filtre.

13. Connecteur selon la revendication 12, dans lequel la sortie expiratoire (16) comprend un ensemble de trous radiaux (55) formés à travers la paroi latérale (5) du corps (2) et communiquant avec le conduit (6) ; et un manchon de connexion (56), formé intégralement avec le corps (2) et s'étendant à partir de la paroi latérale (5) pour entourer l'ensemble des trous (55).

14. Connecteur selon la revendication 12 ou 13, dans lequel la sortie expiratoire (16) est positionnée sur la paroi latérale (5) dans une position angulairement décalée de 90° par rapport à l'entrée MDI (15) et opposée à l'entrée d'oxygène (13) et à la sortie de lecture de débit (14).

15. Connecteur selon l'une des revendications 12 à 14, dans lequel l'entrée d'oxygène (13), la sortie de lecture de débit (14), l'entrée MDI (15) et la sortie expiratoire (16) sont disposées sur trois côtés du corps (2), laissant un quatrième côté sensiblement sans projections définies par des éléments fonctionnels (10).

16. Connecteur selon l'une des revendications précédentes, dans lequel le corps (2) est pourvu d'une ou plusieurs flèches (57) indiquant la direction du flux de ventilation vers le patient, de manière à fournir une indication visuelle de la direction de montage correcte du connecteur (1) par rapport au masque.

17. Connecteur selon l'une des revendications précédentes, dans lequel le corps (2) est constitué d'une pièce monolithique en matériau polymère, par exemple en polycarbonate, et est fait d'un matériau polymère de couleur bleue au moins partiellement transparent, si le connecteur est conçu pour des masques non ventilés ; ou d'un matériau polymère blanc ou non coloré au moins partiellement transparent, si le connecteur est conçu pour des masques ventilés.
